# EUROPÄISCHE PATENTSCHRIFT

(11) **EP 0 794 172 B1**
(45) Veröffentlichungstag und Bekanntmachung des Hinweises auf die Patenterteilung: **24.10.2001**
(21) Anmeldenummer: 97102607.5
(22) Anmeldetag: 19.02.1997
(51) Int. Cl.: C07C 279/22, A61K 31/155

(54) **Ortho-substituierte Benzoylguanidine**
Orthosubstituted benzoylguanidines
Benzoylguanidines orthosubstituées

(30) Priorität: 04.03.1996 DE 19608161
(43) Veröffentlichungstag der Anmeldung: 10.09.1997
(73) Patentinhaber: HOECHST AKTIENGESELLSCHAFT, 65929 Frankfurt am Main (DE)
(72) Erfinder: Albus, Udo, Dr., 61197 Florstadt (DE); Brendel, Joachim, Dr., 61118 Bad Vilbel (DE); Kleemann, Heinz-Werner, Dr., 65474 Bischofsheim (DE); Lang, Hans Jochen, Dr., 65719 Hofheim (DE); Scholz, Wolfgang, Dr., 65760 Eschborn (DE); Schwark, Jan-Robert, Dr., 65929 Frankfurt (DE); Weichert, Andreas, Dr., 63329 Egelsbach (DE)

(56) Entgegenhaltungen:
- EP-A- 0 612 723
- EP-A- 0 640 588
- EP-A- 0 699 666
- EP-A- 0 743 301
- EP-A- 0 760 365
- DE-A- 4 421 495
- DE-A- 4 430 213

## Beschreibung

Die Erfindung betrifft Benzoylguanidine der Formel I 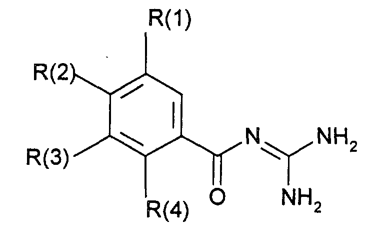 worin bedeuten:
R(1) H, F, Cl, Br, I, CN, NO₂, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CH₂)_{b}-(CF 2)_{c}-CF₃;
X Sauerstoff, S, NR(5),
a Null oder 1;
b Null, 1 oder 2;
c Null, 1, 2 oder 3;
R (5) H, Alkyl mit 1, 2, 3 oder 4 C-Atomen oder -C_{d}H_{2d}R(6);
d Null, 1, 2, 3 oder 4;
R(6) Cycloalkyl mit 3, 4, 5, 6, 7 oder 8 C-Atomen, Phenyl, Biphenylyl oder Naphthyl,
wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(7)R(8);
R(7) und R(8)
unabhängig H oder Alkyl mit 1, 2, 3 oder 4 C-Atomen;
oder
R(1) -SR(10), -OR(10) oder-CR(10)R(11)R(12);
R(10) -C_{f}H_{2f}- Cycloalkyl mit 3, 4, 5, 6, 7 oder 8 C-Atomen im Cycloalkylring, oder Phenyl,
wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
f Null, 1 oder 2;
R(11) und R(12)
unabhängig voneinander wie R(10) definiert oder Wasserstoff oder Alkyl mit 1, 2, 3 oder 4 C-Atomen;
oder
R(1) Phenyl, Naphthyl, Biphenylyl oder Heteroaryl mit 1, 2, 3, 4, 5, 6, 7, 8 oder 9 C-Atomen, letzteres über ein C- oder ein N- Atom des Rings verknüpft,
die jeweils unsubstituiert oder substituiert sind mit 1 - 3 Substituenten ausgewählt aus der Gruppe bestehend aus F, Cl, CF₃ , CH₃, Methoxy, Hydroxy, Amino, Methylamino und Dimethylamino,
oder
R(1) -SR(13), -OR(13), -NHR(13), -NR(13)R(14), -CHR(13)R(15), -C[R(15)R(16)OH], -C=CR(18), -C[R(19)]=CHR(18), -C[R(20)R(21)]ₖ-(CO)-[CR(22)R(23)]_{I}-R(24),
k Null, 1, 2, 3 oder 4;
l Null, 1, 2, 3 oder 4;
R(13) und R(14)
gleich oder verschieden -(CH₂)_{g}-(CHOH)ₕ-(CH₂)ᵢ-(CHOH)ⱼ-R(17) oder -(CH₂)_{g}-O-(CH₂-CH₂O)ₕ-R(24);
R(17) Wasserstoff oder Methyl,
g, h und i
gleich oder verschieden Null, 1, 2, 3 oder 4;
j 1, 2, 3 oder 4;
R(15) und R(16)
gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;
R(18)
Phenyl,
das unsubstituiert oder substituiert ist mit 1 - 3 Substituenten ausgewählt aus der Gruppe bestehend aus F, Cl, CF₃, Methyl, Methoxy und NR(25)R(26);
R(25) und R(26)
H oder Alkyl mit 1, 2, 3 oder 4 C-Atomen;
oder
R(18) Heteroaryl mit 1, 2, 3, 4, 5, 6, 7, 8 oder 9 Kohlenstoffatomen,
das unsubstituiert oder wie Phenyl substituiert ist;
oder
R(18) Alkyl mit 1, 2, 3, 4, 5 oder 6 C-Atomen,
das unsubstituiert oder mit 1 - 3 OH substituiert ist;
oder
R (18)
Cycloalkyl mit 3, 4, 5, 6, 7 oder 8 C-Atomen;
R(19), R(20), R(21), R(22) und R(23)
gleich oder verschieden Wasserstoff oder Methyl;
R(24) H, Alkyl mit 1, 2, 3, 4, 5 oder 6 C-Atomen, Cycloalkyl mit 3, 4, 5, 6, 7 oder 8 C-Atomen oder -Cₘ H₂ₘ-R(18);
m 1, 2, 3 oder 4;
einer der beiden Substituenten R(2) und R(3) Hydroxyl;
und
der jeweils andere der Substituenten R(2) und R(3) definiert ist wie R(1);
R(4) Alkyl mit 1, 2, 3 oder 4 C-Atomen; Alkoxy mit 1, 2, 3 oder 4 C-Atomen, F, Cl, Br, I oder -(CH₂)ₙ-(CF₂)ₒ-CF₃;
n Null oder 1;
o Null oder 1;
sowie deren pharmazeutisch verträgliche Salze.

Bevorzugt sind Verbindungen der Formel I, in denen bedeuten:
R(1) H, F, Cl, Br, I, CN, NO₂, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CF₂)_{c}-CF₃;
X Sauerstoff;
a Null oder 1;
c Null, 1, 2 oder 3;
oder
R(1) -SR(10) oder -OR(10);
R(10) -C_{f}H_{2f}- Cycloalkyl mit 3, 4, 5, 6, 7 oder 8 C-Atomen im Cycloalkylring, oder Phenyl,
wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
f Null oder 1;
oder
R(1) Phenyl, Naphthyl, Biphenylyl oder Heteroaryl mit 1, 2, 3, 4, 5, 6, 7, 8 oder 9 C-Atomen, letzteres über ein C- oder N- Atom des Rings verknüpft,
die unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
oder
R(1) -SR(13), -OR(13), -NHR(13), -NR(13)R(14),-C≡CR(18) oder -C[R(19)]=CHR(18);
R(13) und R(14)
gleich oder verschieden -(CH₂)_{g}-(CHOH)ₕ-(CH₂)ᵢ-(CHOH)ⱼ-R(17) oder -(CH₂)_{g}-O-(CH₂-CH₂O)ₕ-R(24);
R(17) Wasserstoff oder Methyl,
g, h und i
gleich oder verschieden Null, 1 oder 2;
j 1 oder 2;
R(18) Phenyl,
das unsubstituiert oder substituiert ist mit 1 - 3 Substituenten ausgewählt aus der Gruppe bestehend aus F, Cl, CF₃, Methyl, Methoxy und NR(25)R(26);
R(25) und R(26)
H oder Alkyl mit 1, 2, 3 oder 4 C-Atomen;
oder
R(18) Heteroaryl mit 1, 2, 3, 4, 5, 6, 7, 8 oder 9 Kohlenstoffatomen, das unsubstituiert oder wie Phenyl substituiert ist;
oder
R(18) Alkyl mit 1, 2, 3, 4, 5 oder 6 C-Atomen, das unsubstituiert oder mit 1 - 3 OH substituiert ist;
oder
R(18)
Cycloalkyl mit 3, 4, 5, 6, 7 oder 8 C-Atomen;
R(19) Wasserstoff oder Methyl;
einer der Substituenten R(2) und R(3) Hydroxyl;
und
der jeweils andere der Substituenten R(2) und R(3) definiert ist wie R(1);
R(4) Alkyl mit 1 oder 2 C-Atomen, Methoxy, F, Cl, Br, CN oder -(CH₂)ₙ-(CF₂)ₒ-CF_{3;}
n Null oder 1;
o Null oder 1.
sowie deren pharmazeutisch verträgliche Salze.

Besonders bevorzugt sind Verbindungen der Formel I nach Anspruch 1 oder 2, dadurch gekennzeichnet, daß darin bedeuten:
R(1) H, F, Cl, Alkyl mit 1, 2, 3 oder 4 C-Atomen, Alkoxy mit 1, 2, 3 oder 4 C-Atomen, Cycloalkyl mit 5 oder 6 C-Atomen, Cycloalkoxy mit 5 oder 6 C-Atomen oder -Xₐ-(CF₂)_{c}-CF₃;
X Sauerstoff;
a Null oder 1;
c Null oder 1;
oder
R(1) -SR(10) oder -OR(10);
R(10) Cycloalkyl mit 4, 5 oder 6 C-Atomen, oder Phenyl,
wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(1) Chinolyl, Isochinolyl, Pyridyl,
die über ein C- oder N- Atom des Rings verknüpft sind;
und die unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
oder
R(1) Phenyl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino,
oder
R(1) -C≡CR(18);
R(18) Phenyl oder Cycloalkyl mit fünf oder 6 C-Atomen;
einer der beiden Substituenten R(2) und R(3) Hydroxyl;
und der jeweils andere der Substituenten R(2) und R(3) definiert ist wie R(1);
R(4) Methyl, Methoxy, F, Cl oder CF₃.
sowie deren pharmazeutisch verträgliche Salze.

Ganz besonders bevorzugt sind Verbindungen der Formel I, in denen bedeuten:
R(1) H, F, Cl, Alkyl mit 1, 2, 3 oder 4 C-Atomen, Alkoxy mit 1, 2, 3 oder 4 C-Atomen, Cycloalkyl mit 5 oder 6 C-Atomen, Cycloalkoxy mit 5 oder 6 C-Atomen oder CF₃;
einer der Substituenten R(2) und R(3) Hydroxyl,
und
der jeweils andere der Substituenten R(2) und R(3) definiert ist wie R(1);
R(4) Methyl, Methoxy, F, Cl oder CF₃;
sowie deren pharmazeutisch verträgliche Salze

Besonders speziell bevorzugt sind die Verbindungen der Formel I, welche ausgewählt sind aus der Gruppe bestehend aus 2-Chlor-4-hydroxy-benzoylguanidinhydrochlorid, 2-Chlor-4-hydroxy-5-iod-benzoylguanidin-hydrochlorid, 2-Chlor-4-hydroxy-3,5-di-iod-benzoylguanidin-hydrochlorid, 2-Chlor-4-hydroxy-5-trifluormethylbenzoylguanidin-hydrochlorid, 4-Hydroxy-2,5-trifluormethyl-benzoylguanidinhydrochlorid und 4-Hydroxy-2-methyl-5-trifluormethyl-benzoylguanidin-hydrochlorid.

Unter Heteroaryl mit 1, 2, 3, 4, 5, 6, 7, 8 oder 9 C-Atomen werden Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl mit 1, 2, 3, 4, 5, 6, 7, 8 oder 9 C-Atomen gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl; besonders Furanyl, Thienyl, Pyrrolyl, Imidazolyl, Pyrazolyl, Triazolyl, Thiazolyl, Pyridyl, Indolyl, Chinolyl und Isochinolyl.

Enthält einer der Substituenten R(1) bis R(4) ein oder mehrere Asymmetriezentren, so können diese sowohl S als auch R konfiguriert sein. Die Verbindungen können als optische Isomere, als Diastereomere, als Racemate oder als Gemische derselben vorliegen.

Die bezeichneten Alkylreste können geradkettig oder verzweigt sein.

Zur Herstellung der Verbindung I setzt man eine Verbindung der Formel II 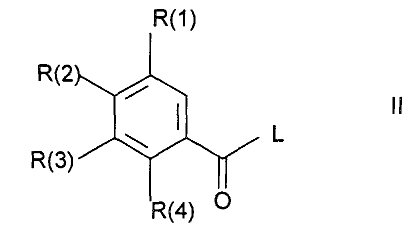 mit Guanidin um worin R(1) bis R(4) die angegebene Bedeutung besitzen und L für eine leicht nucleophil substituierbare leaving group steht.

Die aktivierten Säurederivate der Formel II, worin L eine Alkoxy-, vorzugsweise eine Methoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zu Grunde liegenden Carbonsäurechloriden (Formel II, L = Cl), die man ihrerseits wiederum in an sich bekannter Weise aus den zu Grunde liegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zu Grunde liegenden Benzoesäure-derivaten (Formel II, L = OH) herstellen, wie die Methylester der Formel II mit L = OCH₃ durch Behandeln mit gasförmigem HCI in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)methylen)amino]-1,1,3,3-tetramethyluron ium-tetrafluorborat ("TOTU")[Weiss und Krommer, Chemiker Zeitung 98, 817 (1974)]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20□C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Ein Teil der zu Grunde liegenden Benzoesäurederivate der Formel II ist bekannt und in der Literatur beschrieben. Die unbekannten Verbindungen der Formel II können nach literaturbekannten Methoden hergestellt werden. Die erhaltenen Benzoesäuren werden nach einer der oben beschriebenen Verfahrensvarianten zu erfindungsgemäßen Verbindungen I umgesetzt.

Die Einführung einiger Substituenten in 2-, 3-, 4- und 5-Stellung gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. zinkverbindungen.

Benzoylguanidine I sind im allgemeinen schwache Basen und können Säure unter Bildung von Salzen binden. Als Säureadditionssalze kommen Salze aller pharmakologisch verträglichen Säuren in frage, beispielsweise Halogenide, insbesondere Hydrochloride, Lactate, Sulfate, Citrate, Tartrate, Acetate, Phosphate, Methylsulfonate, p-Toluolsulfonate.

Die Verbindungen I sind substituierte Acylguanidine.

Die EP 640 588 und EP 612 723 sowie die DE 44 30 213 beschreiben ähnliche Verbindungen, jedoch keine mit gleichzeitig einer Hydroxylgruppe unter R2 und R3 sowie der Substitution in Ortho- Stellung, nämlich R(4) als Alkyl, Alkoxy, Halogen oder CF₃.

Gegenüber den bekannten Verbindungen zeichnen sich die erfindungsgemäßen Verbindungen durch eine außerordentlich hohe Wirksamkeit in der Inhibition des Na⁺/H⁺- Austauschs aus, sowie durch eine verbesserte Wasserlöslichkeit aus

Sie haben ebenso wie die bekannten Verbindungen keine unerwünschten und nachteiligen salidiuretischen, jedoch sehr gute antiarrhythmische Eigenschaften, wie sie beispielsweise zur Behandlung von Krankheiten wichtig sind, die bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z. B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien- Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässerige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- MeOH: Methanol
- DMF: N,N-Dimethylformamid
- RT: Raumtemperatur
- EE: Ethylacetat (EtOAc)
- Smp: Schmelzpunkt
- THF: Tetrahydrofuran
- eq.: Äquivalent

### Experimenteller Teil

### Allgemeine Vorschrift zur Herstellung von Benzoyl-guanidinen (I)

### Variante A: aus Benzoesäuren (II, L = OH)

1.0 eq. des Benzoesäurederivates der Formel II löst bzw. suspendiert man in wasserfreiem THF ( 5 ml/mmol) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck (Rotationsverdampfer) ab, versetzt mit Wasser, stellt mit 2 N HCI auf pH 6 bis 7 und filtriert das entsprechende Benzoylguanidin (Formel I) ab. Die so erhaltenen Benzoylguanidine können durch Behandeln mit wässeriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I)

### Variante B: aus Benzoesäure-alkylestern (II, L = O-Alkyl)

1.0 eq. des Benzoesäure-alkylesters der Formel II sowie 5.0 eq. Guanidin (freie Base) werden in Isopropanol gelöst oder in THF suspendiert und bis zum vollständigen Umsatz (Dünnschichtkontrolle) zum Sieden erhitzt (typische Reaktionszeit 2 bis 5 h). Das Lösungsmittel wird unter vermindertem Druck (Rotationsverdampfer) abdestilliert in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Beispiel 1: 2-Chlor-4-hydroxy-benzoylguanidin-hydrochlorid

Farblose Kristalle,
Smp. 247°C
aus 2-Chlor-4-hydroxy-benzoesäure nach Variante A.

### Beispiel 2: 2-Chlor-4-hydroxy-5-iod-benzoylguanidin-hydrochlorid

Farblose Kristalle,
Smp. 246 - 47°C

### Syntheseweg:

a) 2-Chlor-4-hydroxy-benzoesäuremethylester aus 2-Chlor-4-hydroxy-benzoesäure durch Veresterung mit Methanol (Überschuß) in Gegenwart von 10 Äquivalenten Acetylchlorid innerhalb 24 h bei RT. Nach wässeriger Aufarbeitung erfolgt Kristallisation aus Ether/Cyclohexan,
   farblose Kristalle,
   Smp. 128 - 30°
b) 2-Chlor-4-hydroxy-5-iod-benzoesäuremethylester aus a) durch Reaktion mit einem Gemisch aus 1.1 Äquivalenten N-Chlorsuccinimid und 2.1 Äquivalenten Kaliumiodid in Gegenwart von 10 Äquivalenten Natriumacetat in halbkonzentriertem Eisessig bei RT innerhalb von 1.5 h. Wässerige Aufarbeitung und Säulenchromatographie mit Cyclohexan/Essigester 9 : 1 liefert das gewünschte Produkt als farblosen Feststoff,
   Smp. 191 - 92°C,
   aber auch 2-Chlor-4-hydroxy-3,5-di-iod-benzoesäuremethylester,
   farbloser Feststoff,
   Smp. 131 - 32°C.
c) 2-Chlor-4-hydroxy-5-iod-benzoylguanidin-hydrochlorid aus 2-Chlor-4-hydroxy-5-iod-benzoesäuremethylester (vgl. 2 b) nach Variante B.

### Beispiel 3: 2-Chlor-4-hydroxy-3,5-di-iod-benzoylguanidin-hydrochlorid

Farbloser Feststoff,
Smp. 205 - 10°C (Zersetzung),
aus 2-Chlor-4-hydroxy-3,5-di-iod-benzoesäuremethylester (vgl. 2 b) nach Verfahren B.

### Beispiel 4: 2-Chlor-4-hydroxy-5-trifluormethyl-benzoylguanidin-hydrochlorid

Farbloser Feststoff,
amorph, (M+H)⁺: 282

### Syntheseweg:

a) 2-Chlor-4-benzyloxy-5-iod-benzoesäuremethylester aus 2-Chlor-4-hydroxy-5-iod-benzoesäuremethylester durch Reaktion mit 1.1 Äquivalenten Benzylbromid in Gegenwart von Kaliumkarbonat in DMF bei 80°C innerhalb 3 h. Nach wässeriger Aufarbeitung wird aus Isopropanol umkristallisiert,
   farblose Kristalle,
   Smp. 102 - 08°C.
b) 2-Chlor-4-benzyloxy-5-trifluormethyl-benzoesäuremethylester aus 4 a) durch Erhitzen auf 160°C mit 2 Äquivalenten Kaliumtrifluoracetat in N-Methylpyrrolid-2-on in Gegenwart von Kupfer(I)iodid innerhalb 5 h. Wässerige Aufarbeitung und Säulenchromatographie mit Cyclohexan/Essigester 9 : 1 liefert farblose Kristalle, Smp. 126 - 27°C.
c) 2-Chlor-4-benzyloxy-5-trifluormethyl-benzoylguanidin aus 4 b) nach Verfahren
   B ohne Salzbildung,
   farblose Kristalle,
   Smp. 180°C
d) 2-Chlor-4-hydroxy-5-trifluormethyl-benzoylguanidin-hydrochlorid aus 4 c) durch Hydrierung mittels Palladium/Kohle und anschließender Hydrochloridbildung,
   farbloser Feststoff, amorph, (M+H)⁺: 282.

### Beispiel 5: 4-Hydroxy-2,5-trifluormethyl-benzoylguanidin-hydrochlorid:

Farblose Kristalle,
Smp. 211°C.

### Syntheseweg:

a) 4-Benzyloxy-2,5-trifluormethyl-benzoesäuremethylester
   aus 4 b) durch analoge Trifluormethylierung, jedoch bei erhöhter Temperatur (180°C) und einer Reaktionszeit von 5 h,
   farblose Kristalle,
   Smp.116°C.
b) 4-Benzyloxy-2,5-trifluormethyl-benzoylguanidin-hydrochlorid
   aus 5 a) nach der allgemeinen Vorschrift,
   farblose Kristalle,
   Smp. 221°C.
c) 4-Hydroxy-2,5-trifluormethyl-benzoylguanidin-hydrochlorid
   aus 5 b) durch Hydrierung an 10%-Palladium/Kohle in Methanol bei RT.

### Beispiel 6: 4-Hydroxy-2-methyl-5-trifluormethyl-benzoylguanidin-hydrochlorid: Amorpher Festkörper.

Syntheseweg:
a) 2-Methyl-4-benzyloxy-5-trifluormethyl-benzoesäuremethylester
   aus 2-Chlor-4-benzyloxy-5-trifluormethylbenzoesäuremethylester (4 b) via cross-coupling mit 1.2 Äquivalenten Methylzinkchlorid (aus Methylmagnesiumchlorid durch Transmetallierung mit Zink(II)chlorid-THF-Komplex) durch Rühren bei 80°C in DMF in Gegenwart katalytischen Mengen an Palladium(II)acetat und Kupfer(I)iodid,
   farblose Kristalle,
   Smp. 105°C.
b) 4-Benzyloxy-2-methyl-5-trifluormethyl-benzoylguanidin-hydrochlorid
   aus 6 a) nach der allgemeinen Vorschrift,
   farblose Kristalle,
   Smp. 255°C.
c) 4-Hydroxy-2-methyl-5-trifluormethyl-benzoylguanidin-hydrochlorid
   aus 6 b) analog 5 c).

### Pharmakologische Daten:

Inhibition des Na⁺/H⁺-Exchangers von Kaninchenerythrocyten Weiße Neuseeland-Kaninchen (Ivanovas) erhielten eine Standard-Diät mit 2% Cholesterin für sechs Wochen, um den Na⁺/H⁺-Austausch zu aktivieren und so den Na⁺-Influx in die Erythrocyten via Na⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-Influx zu bestimmen, wurden 100 µl jeder Blutprobe in jeweils 5 ml eines hyperosmolaren Salz-Sucrose-Mediums (mmol/l: 140 NaCI, 3 KCI, 150 Sucrose, 0,1 Ouabain, 20 Tris-hydroxymethylaminomethan) bei pH 7,4 und 37C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amilorid-hemmbare Natrium-Influx ergab sich aus der Differenz des Natriumgehalts der Erythrocyten nach Inkubation mit und ohne Amilorid 3 x 10⁻⁴ mol/l. Auf diese Weise wurde auch bei den erfindungsgemäßen Verbindungen verfahren.

### Ergebnisse

| Inhibition des Na⁺/H⁺-Exchangers: | |
|---|---|
| Beispiel | IC₅₀(mol/l) |
| 1 | 4.0 x 10⁻⁶ |
| 2: | 0.24 x 10⁻⁶ |
| 3: | 0.10 x 10⁻⁶ |
| 4: | 0.14 x 10⁻⁶ |

## Patentansprüche

1. Ortho-substituierte Benzoylguanidine der Formel I 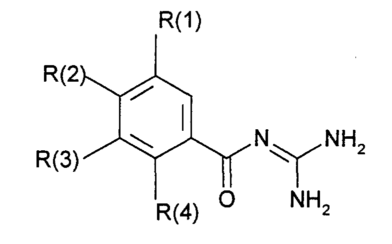 worin bedeuten:
R(1) H, F, Cl, Br, I, CN, NO₂, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CH₂)_{b}-(CF₂)_{c}-CF₃;
X Sauerstoff, S, NR(5),
a Null oder 1;
b Null, 1 oder 2;
c Null, 1, 2 oder 3;
R (5) H, Alkyl mit 1, 2, 3 oder 4 C-Atomen oder -C_{d}H_{2d}R(6);
d Null, 1, 2, 3 oder 4;
R(6) Cycloalkyl mit 3, 4, 5, 6, 7 oder 8 C-Atomen, Phenyl, Biphenylyl oder Naphthyl,
wobei die Aromaten Phenyl, Biphenylyl oder Naphthyl nicht substituiert oder substituiert sind mit 1 - 3 Substituenten ausgewählt aus der Gruppe bestehend aus F, Cl, CF₃, Methyl, Methoxy und NR(7)R(8);
R(7) und R(8)
unabhängig H oder Alkyl mit 1, 2, 3 oder 4 C-Atomen;
oder
R(1) -SR(10), -OR(10) oder -CR(10)R(11)R(12);
R(10) -C_{f}H_{2f}- Cycloalkyl mit 3, 4, 5, 6, 7 oder 8 C-Atomen im Cycloalkylring, oder Phenyl,
wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
f Null, 1 oder 2;
R(11) und R(12)
unabhängig voneinander wie R(10) definiert oder Wasserstoff oder Alkyl mit 1, 2, 3 oder 4 C-Atomen;
oder
R(1) Phenyl, Naphthyl, Biphenylyl oder Heteroaryl mit 1, 2, 3, 4, 5, 6, 7, 8 oder 9 C-Atomen, letzteres über ein C- oder ein N- Atom des Rings verknüpft,
die jeweils unsubstituiert oder substituiert sind mit 1 - 3 Substituenten ausgewählt aus der Gruppe bestehend aus F, Cl, CF 3, CH₃, Methoxy, Hydroxy, Amino, Methylamino und Dimethylamino,
oder
R(1) -SR(13), -OR(13), -NHR(13), -NR(13)R(14), -CHR(13)R(15), -C[R(15)R(16)OH], -C≡CR(18), -C[R(19)]=CHR(18), -C[R(20)R(21)]ₖ-(CO)-[CR(22)R(23)]ₗ-R(24),
k Null, 1, 2, 3 oder 4;
l Null, 1, 2, 3 oder 4;
R(13) und R(14)
gleich oder verschieden -(CH₂)_{g}-(CHOH)ₕ-(CH₂)ᵢ-(CHOH)ⱼ-R(17) oder -(CH₂)_{g}-O-(CH₂-CH₂O)ₕ-R(24);
R(17) Wasserstoff oder Methyl,
g, h und i
gleich oder verschieden Null, 1, 2, 3 oder 4;
j 1, 2, 3 oder 4;
R(15) und R(16)
gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;
R(18)
Phenyl,
das unsubstituiert oder substituiert ist mit 1 - 3 Substituenten ausgewählt aus der Gruppe bestehend aus F, Cl, CF₃, Methyl, Methoxy und NR(25)R(26);
R(25) und R(26)
H oder Alkyl mit 1, 2, 3 oder 4 C-Atomen;
oder
R(18) Heteroaryl mit 1, 2, 3, 4, 5, 6, 7, 8 oder 9 Kohlenstoffatomen, das unsubstituiert oder wie Phenyl substituiert ist;
oder
R(18) Alkyl mit 1, 2, 3, 4, 5 oder 6 C-Atomen, das unsubstituiert oder mit 1 - 3 OH substituiert ist;
oder
R (18)
Cycloalkyl mit 3, 4, 5, 6, 7 oder 8 C-Atomen;
R(19), R(20), R(21), R(22) und R(23)
gleich oder verschieden Wasserstoff oder Methyl;
R(24) H, Alkyl mit 1, 2, 3, 4, 5 oder 6 C-Atomen, Cycloalkyl mit 3, 4, 5, 6, 7 oder 8 C-Atomen oder -CₘH₂ₘ-R(18);
m 1, 2, 3 oder 4;
einer der beiden Substituenten R(2) und R(3)
Hydroxyl;
und
der jeweils andere der Substituenten R(2) und R(3) definiert ist wie R(1);
R(4) Alkyl mit 1, 2, 3 oder 4 C-Atomen; Alkoxy mit 1, 2, 3 oder 4 C-Atomen, F, Cl, Br, I oder -(CH₂)ₙ-(CF₂)ₒ-CF₃;
n Null oder 1;
o Null oder 1;
sowie deren pharmazeutisch verträgliche Salze.

2. Verbindungen der Formel I nach Anspruch 1, **dadurch gekennzeichnet, daß** darin bedeuten:
R(1) H, F, Cl, Br, I, CN, NO₂, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder Xₐ-(CF₂)_{c}-CF₃;
X Sauerstoff;
a Null oder 1;
c Null, 1, 2 oder 3;
oder
R(1) -SR(10) oder -OR(10);
R(10) -C_{f}H_{2f}- Cycloalkyl mit 3, 4, 5, 6, 7 oder 8 C-Atomen im Cycloalkylring, oder Phenyl,
wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
f Null oder 1;
oder
R(1) Phenyl, Naphthyl, Biphenylyl oder Heteroaryl mit 1, 2, 3, 4, 5, 6, 7, 8 oder 9 C-Atomen, letzteres über ein C- oder N- Atom des Rings verknüpft,
die unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
oder
R(1) -SR(13), -OR(13), -NHR(13), -NR(13)R(14),-C≡CR(18) oder -C[R(19)]=CHR(18);
R(13) und R(14)
gleich oder verschieden -(CH₂)_{g}-(CHOH)ₕ-(CH₂)ᵢ-(CHOH)ⱼ-R(17) oder -(CH₂)_{g}-O-(CH₂-CH₂O)ₕ-R(24);
R(17) Wasserstoff oder Methyl,
g, h und i
gleich oder verschieden Null, 1 oder 2;
j 1 oder 2;
R(18)
Phenyl,
das unsubstituiert oder substituiert ist mit 1 - 3 Substituenten ausgewählt aus der Gruppe bestehend aus F, Cl, CF₃, Methyl, Methoxy und NR(25)R(26);
R(25) und R(26)
H oder Alkyl mit 1, 2, 3 oder 4 C-Atomen;
oder
R(18) Heteroaryl mit 1, 2, 3, 4, 5, 6, 7, 8 oder 9 Kohlenstoffatomen, das unsubstituiert oder wie Phenyl substituiert ist;
oder
R(18) Alkyl mit 1, 2, 3, 4, 5 oder 6 C-Atomen, das unsubstituiert oder mit 1 - 3 OH substituiert ist;
oder
R (18)
Cycloalkyl mit 3, 4, 5, 6, 7 oder 8 C-Atomen;
R(19) Wasserstoff oder Methyl;
einer der Substituenten R(2) und R(3) Hydroxyl;
und
der jeweils andere der Substituenten R(2) und R(3) definiert ist wie R(1);
R(4) Alkyl mit 1 oder 2 C-Atomen, Methoxy, F, Cl, Br, CN oder -(CH₂)ₙ-(CF₂)ₒ-CF₃;
n Null oder 1;
o Null oder 1.

3. Verbindungen der Formel I nach Anspruch 1 oder 2, **dadurch gekennzeichnet, daß** darin bedeuten:
R(1) H, F, Cl, Alkyl mit 1, 2, 3 oder 4 C-Atomen, Alkoxy mit 1, 2, 3 oder 4 C-Atomen, Cycloalkyl mit 5 oder 6 C-Atomen, Cycloalkoxy mit 5 oder 6 C-Atomen oder -Xₐ-(CF₂)_{c}-CF₃;
X Sauerstoff;
a Null oder 1;
c Null oder 1;
oder
R(1) -SR(10) oder -OR(10);
R(10) Cycloalkyl mit 4, 5 oder 6 C-Atomen, oder Phenyl,
wobei Phenyl unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino;
oder
R(1) Chinolyl, Isochinolyl, Pyridyl,
die über ein C- oder N- Atom des Rings verknüpft sind; und die unsubstituiert oder substituiert sind mit 1 - 3 Substituenten ausgewählt aus der Gruppe bestehend aus F, Cl, CF₃, CH₃, Methoxy, Hydroxy, Amino, Methylamino und Dimethylamino,
oder
R(1) Phenyl,
das unsubstituiert oder substituiert ist mit 1 - 3 Substituenten ausgewählt aus der Gruppe bestehend aus F, Cl, CF₃, CH₃, Methoxy, Hydroxy, Amino, Methylamino und Dimethylamino,
oder
R(1) -C≡CR(18);
R(18)
Phenyl oder Cycloalkyl mit fünf oder 6 C-Atomen;
einer der beiden Substituenten R(2) und R(3) Hydroxyl;
und der jeweils andere der Substituenten R(2) und R(3) definiert ist wie R(1);
R(4) Methyl, Methoxy, F, Cl oder CF₃.

4. Verbindungen der Formel I nach einem oder mehreren der Ansprüche 1 bis 3, **dadurch gekennzeichnet, daß** darin bedeuten:
R(1) H, F, Cl, Alkyl mit 1, 2, 3 oder 4 C-Atomen, Alkoxy mit 1, 2, 3 oder 4 C-Atomen, Cycloalkyl mit 5 oder 6 C-Atomen, Cycloalkoxy mit 5 oder 6 C-Atomen oder CF₃;
einer der Substituenten R(2) und R(3) Hydroxyl,
und
der jeweils andere der Substituenten R(2) und R(3) definiert ist wie R(1);
R(4) Methyl, Methoxy, F, Cl oder CF₃.

5. Verbindung der Formel I nach Anspruch 1, ausgewählt aus der Gruppe bestehend aus : 2-Chlor-4-hydroxy-benzoylguanidin-hydrochlorid, 2-Chlor-4-hydroxy-5-iod-benzoylguanidin-hydrochlorid, 2-Chlor-4-hydroxy-3,5-di-iod-benzoylguanidin-hydrochlorid, 2-Chlor-4-hydroxy-5-trifluormethyl-benzoylguanidin-hydrochlorid, 4-Hydroxy-2,5-trifluormethyl-benzoylguanidin-hydrochlorid und 4-Hydroxy-2-methyl-5-trifluormethyl-benzoylguanidin-hydrochlorid.

6. Verwendung einer Verbindung I nach Anspruch 1 zur Herstellung eines Medikaments zur Behandlung oder Prophylaxe von durch ischämische Zustände bewirkten Krankheiten.

7. Verwendung einer Verbindung I nach Anspruch 1 zur Herstellung eines Medikaments zur Behandlung oder Prophylaxe des Herzinfarkts.

8. Verwendung einer Verbindung I nach Anspruch 1 zur Herstellung eines Medikaments zur Behandlung oder Prophylaxe der Angina Pectoris.

9. Verwendung einer Verbindung I nach Anspruch 1 zur Herstellung eines Medikaments zur Behandlung oder Prophylaxe von ischämischen Zuständen des Herzens.

10. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

11. Verwendung einer Verbindung I nach Anspruch 1 zur Herstellung eines Medikaments zur Behandlung oder Prophylaxe von ischämischen Zuständen peripherer Organe und Gliedmaßen.

12. Verwendung einer Verbindung I nach Anspruch 1 zur Herstellung eines Medikaments zur Behandlung von Schockzuständen.

13. Verwendung einer Verbindung I nach Anspruch 1 zur Herstellung eines Medikaments zum Einsatz bei chirurgischen Operationen und Organtransplantationen.

14. Verwendung einer Verbindung I nach Anspruch 1 zur Herstellung eines Medikaments zur Konservierung und Lagerung von Transplantaten für chirurgische Maßnahmen.

15. Verwendung einer Verbindung I nach Anspruch 1 zur Herstellung eines Medikaments zur Behandlung von Krankheiten, bei denen die Zellproliferation eine primäre oder sekundäre Ursache darstellt.

16. Arzneimittel, **gekennzeichnet durch** einen wirksamen Gehalt einer Verbindung der Formel I nach Ansprüchen 1 bis 5.

## Claims

1. An ortho-substituted benzoylguanidine of the formula I 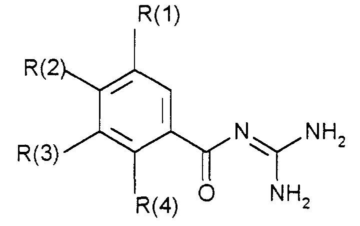 in which:
R(1) is H, F, Cl, Br, I, CN, NO₂, alkyl having 1, 2, 3, 4, 5, 6, 7 or 8 carbon atoms, alkoxy having 1, 2, 3, 4, 5, 6, 7 or 8 carbon atoms, cycloalkyl having 3, 4, 5, 6, 7 or 8 carbon atoms, cycloalkoxy having 3, 4, 5, 6, 7 or 8 carbon atoms or Xₐ-(CH₂)_{b}-(CF₂)_{c}-CF₃;
X is oxygen, S or NR(5),
a is zero or 1;
b is zero, 1 or 2;
c is zero, 1, 2 or 3;
R (5) is H, alkyl having 1, 2, 3 or 4 carbon atoms or -C_{d}H_{2d}R(6);
d is zero, 1, 2, 3 or 4;
R(6) is cycloalkyl having 3, 4, 5, 6, 7 or 8 carbon atoms, phenyl, biphenylyl or naphthyl,
where the aromatic radicals phenyl, biphenylyl or naphthyl are unsubstituted or substituted by 1 - 3 substituents selected from the group consisting of F, Cl, CF₃, methyl, methoxy and NR(7)R(8);
R(7) and R(8) are,
independently, H or alkyl having 1, 2, 3 or 4 carbon atoms;
or
R(1) is -SR(10), -OR(10) or -CR(10)R(11)R(12);
R(10) is -C_{f}H_{2f}-cycloalkyl having 3, 4, 5, 6, 7 or 8 carbon atoms in the cycloalkyl ring, or phenyl,
where phenyl is unsubstituted or substituted by 1 - 3 substituents selected from the group consisting of F, Cl, CF₃, CH₃, methoxy, hydroxyl, amino, methylamino and dimethylamino;
f is zero, 1 or 2;
R(11) and R(12) are,
independently of each other, defined as R(10), or hydrogen or alkyl having 1, 2, 3 or 4 carbon atoms;
or
R(1) is phenyl, naphthyl, biphenylyl or heteroaryl having 1, 2, 3, 4, 5, 6, 7, 8 or 9 carbon atoms, with the latter being linked by a C atom or an N atom of the ring,
which are in each case unsubstituted or substituted by 1 - 3 substituents selected from the group consisting of F, Cl, CF₃, CH₃, methoxy, hydroxyl, amino, methylamino and dimethylamino,
or
R(1) is -SR(13), -OR(13), -NHR(13), -NR(13)R(14), -CHR(13)R(15), -C[R(15)R(16)OH], -C≡CR(18), -C[R(19)]=CHR(18) or -C[R(20)R(21)]ₖ-(CO)-[CR(22)R(23)]ₗ-R(24),
k is zero, 1, 2, 3 or 4;
l is zero, 1, 2, 3 or 4;
R(13) and R(14) are,
identically or differently, -(CH₂)_{g}-(CHOH)ₕ-(CH₂)ᵢ-(CHOH)ⱼ-R(17) or -(CH₂)_{g}-O-(CH₂-CH₂O)ₕ-R(24);
R(17) is hydrogen or methyl,
g, h and i are, identically or differently, zero, 1, 2, 3 or 4;
j is 1, 2, 3 or 4;
R(15) and R(16) are,
identically or differently, hydrogen, alkyl having 1, 2, 3, 4, 5 or 6 carbon atoms, or are, together with the carbon atom carrying them, cycloalkyl having 3, 4, 5, 6, 7 or 8 carbon atoms;
R(18) is
phenyl,
which is unsubstituted or substituted by 1 - 3 substituents selected from the group consisting of F, Cl, CF₃, methyl, methoxy and NR(25)R(26);
R(25) and R(26) are H or alkyl having 1, 2, 3 or 4 carbon atoms;
or
R(18) is heteroaryl having 1, 2, 3, 4, 5, 6, 7, 8 or 9 carbon atoms, which is unsubstituted or substituted as phenyl;
or
R(18) is alkyl having 1, 2, 3, 4, 5 or 6 carbon atoms, which is unsubstituted or substituted by 1 - 3 OH;
or
R (18) is cycloalkyl having 3, 4, 5, 6, 7 or 8 carbon atoms;
R(19), R(20), R(21), R(22) and R(23) are, identically or differently, hydrogen or methyl;
R(24) is H, alkyl having 1, 2, 3, 4, 5 or 6 carbon atoms, cycloalkyl having 3, 4, 5, 6, 7 or 8 carbon atoms or -CₘH₂ₘ-R(18);
m is 1, 2, 3 or 4;
one of the two substituents R(2) and R(3) is hydroxyl;
and
the other of the substituents R(2) and R(3) in each case is defined as R(1);
R(4) is alkyl having 1, 2, 3 or 4 carbon atoms; alkoxy having 1, 2, 3 or 4 carbon atoms, F, Cl, Br, I or -(CH₂)ₙ-(CF₂)ₒ-CF₃;
n is zero or 1;
o is zero or 1;
and the pharmaceutically tolerated salts thereof.

2. A compound of the formula I as claimed in claim 1, wherein:
R(1) is H, F, Cl, Br, I, CN, NO₂, alkyl having 1, 2, 3, 4, 5, 6, 7 or 8 carbon atoms, alkoxy having 1, 2, 3, 4, 5, 6, 7 or 8 carbon atoms, cycloalkyl having 3, 4, 5, 6, 7 or 8 carbon atoms, cycloalkoxy having 3, 4, 5, 6, 7 or 8 carbon atoms or Xₐ-(CF₂)_{c}-CF₃;
X is oxygen;
a is zero or 1;
c is zero, 1, 2 or 3;
or
R(1) is -SR(10) or -OR(10);
R(10) is -C_{f}H_{2f}-cycloalkyl having 3, 4, 5, 6, 7 or 8 carbon atoms in the cycloalkyl ring, or phenyl,
where phenyl is unsubstituted or substituted by 1 - 3 substituents selected from the group consisting of F, Cl, CF₃, CH₃, methoxy, hydroxyl, amino, methylamino and dimethylamino;
f is zero or 1;
or
R(1) is phenyl, naphthyl, biphenylyl or heteroaryl having 1, 2, 3, 4, 5, 6, 7, 8 or 9 carbon atoms, with the latter being linked via a C atom or N atom of the ring,
which are unsubstituted or substituted by 1 - 3 substituents selected from the group consisting of F, Cl, CF₃, CH₃, methoxy, hydroxyl, amino, methylamino and dimethylamino,
or
R(1) is -SR(13), -OR(13), -NHR(13), -NR(13)R(14),-C≡CR(18) or -C[R(19)]=CHR(18);
R(13) and R(14) are,
identically of differently, -(CH₂)_{g}-(CHOH)ₕ-(CH₂)ᵢ-(CHOH)ⱼ-R(17) or -(CH₂)_{g}-O-(CH₂-CH₂O)ₕ-R(24);
R(17) is hydrogen or methyl,
g, h and i are, identically or differently, zero, 1 or 2;
j is 1 or 2;
R(18) is
phenyl,
which is unsubstituted or substituted by 1 - 3 substituents selected from the group consisting of F, Cl, CF₃, methyl, methoxy and NR(25)R(26);
R(25) and R(26) are
H or alkyl having 1, 2, 3 or 4 carbon atoms;
or
R(18) is heteroaryl having 1, 2, 3, 4, 5, 6, 7, 8 or 9 carbon atoms, which is unsubstituted or substituted as phenyl;
or
R(18) is alkyl having 1, 2, 3, 4, 5 or 6 carbon atoms, which is unsubstituted or substituted by 1 - 3 OH;
or
R (18) is cycloalkyl having 3, 4, 5, 6, 7 or 8 carbon atoms;
R(19) is hydrogen or methyl;
one of the substituents R(2) and R(3) is hydroxyl;
and
the other of the substituents R(2) and R(3) in each case is defined as R(1);
R(4) is alkyl having 1 or 2 carbon atoms, methoxy, F, Cl, Br, CN or -(CH₂)ₙ-(CF₂)ₒ-CF₃;
n is zero or 1;
o is zero or 1.

3. A compound of the formula I as claimed in claim 1 or 2, wherein:
R(1) is H, F, Cl, alkyl having 1, 2, 3 or 4 carbon atoms, alkoxy having 1, 2, 3 or 4 carbon atoms, cycloalkyl having 5 or 6 carbon atoms, cycloalkoxy having 5 or 6 carbon atoms or -Xₐ-(CF₂)_{c}-CF₃;
X is oxygen;
a is zero or 1;
c is zero or 1;
or
R(1) is -SR(10) or -OR(10);
R(10) is cycloalkyl having 4, 5 or 6 carbon atoms, or phenyl,
where phenyl is unsubstituted or substituted by 1 - 3 substituents selected from the group consisting of F, Cl, CF₃, CH₃, methoxy, hydroxyl, amino, methylamino and dimethylamino;
or
R(1) is quinolyl, isoquinolyl or pyridyl,
which are linked via a C atom or N atom of the ring;
and which are unsubstituted or substituted by 1 - 3 substituents selected from the group consisting of F, Cl, CF₃, CH₃, methoxy, hydroxyl, amino, methylamino and dimethylamino,
or
R(1) is phenyl,
which is unsubstituted or substituted by 1 - 3 substituents selected from the group consisting of F, Cl, CF₃, CH₃, methoxy, hydroxyl, amino, methylamino and dimethylamino,
or
R(1) is -C≡CR(18);
R(18) is phenyl or cycloalkyl having five or 6 carbon atoms; one of the two substituents R(2) and R(3) is hydroxyl;
and the other of the substituents R(2) and R(3) in each case is defined as R(1);
R(4) is methyl, methoxy, F, Cl or CF₃.

4. A compound of the formula I as claimed in one or more of claims 1 to 3, wherein:
R(1) is H, F, Cl, alkyl having 1, 2, 3 or 4 carbon atoms, alkoxy having 1, 2, 3 or 4 carbon atoms, cycloalkyl having 5 or 6 carbon atoms, cycloalkoxy having 5 or 6 carbon atoms or CF₃;
one of the substituents R(2) and R(3) is hydroxyl,
and
the other of the substituents R(2) and R(3) in each case is defined as R(1);
R(4) is methyl, methoxy, F, Cl or CF₃.

5. A compound of the formula I as claimed in claim 1, selected from the group consisting of:
2-chloro-4-hydroxybenzoylguanidine hydrochloride,
2-chloro-4-hydroxy-5-iodobenzoylguanidine hydrochloride,
2-chloro-4-hydroxy-3,5-diiodobenzoylguanidine hydrochloride,
2-chloro-4-hydroxy-5-trifluoromethylbenzoylguanidine hydrochloride,
4-hydroxy-2,5-trifluoromethylbenzoylguanidine hydrochloride,
and
4-hydroxy-2-methyl-5-trifluoromethylbenzoylguanidine hydrochloride.

6. The use of a compound I as claimed in claim 1 for preparing a medicament for the treatment or prophylaxis of diseases which are brought about by ischemic conditions.

7. The use of a compound I as claimed in claim 1 for preparing a medicament for the treatment or prophylaxis of cardiac infarction.

8. The use of a compound I as claimed in claim 1 for preparing a medicament for the treatment or prophylaxis of angina pectoris.

9. The use of a compound I as claimed in claim 1 for preparing a medicament for the treatment or prophylaxis of ischemic conditions of the heart.

10. The use of a compound I as claimed in claim 1 for preparing a medicament for the treatment or prophylaxis of ischemic conditions of the peripheral and central nervous system and of stroke.

11. The use of a compound I as claimed in claim 1 for preparing a medicament for the treatment or prophylaxis of ischemic conditions of peripheral organs and limbs.

12. The use of a compound I as claimed in claim 1 for preparing a medicament for the treatment of shock conditions.

13. The use of a compound I as claimed in claim 1 for preparing a medicament for use in surgical operations and organ transplantations.

14. The use of a compound I as claimed in claim 1 for preparing a medicament for the preservation and storage of transplants for surgical procedures.

15. The use of a compound I as claimed in claim 1 for preparing a medicament for the treatment of diseases in which cell proliferation constitutes a primary or secondary cause.

16. A pharmaceutical, which comprises an effective amount of a compound of the formula I as claimed in claims 1 to 5.

## Revendications

1. Benzoylguanidines ortho-substituées de formule I 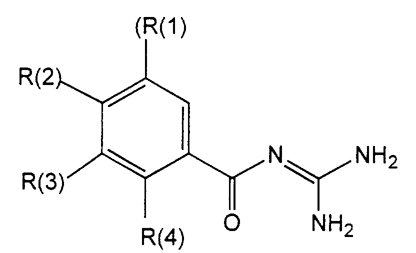 dans laquelle :
R(1) est H, F, Cl, Br, I, CN, NO₂, un radical alkyle ayant 1, 2, 3, 4, 5, 6, 7 ou 8 atomes de carbone, alcoxy ayant 1, 2, 3, 4, 5, 6, 7 ou 8 atomes de carbone, cycloalkyle ayant 3, 4, 5, 6, 7 ou 8 atomes de carbone, cycloalcoxy ayant 3, 4, 5, 6, 7 ou 8 atomes de carbone ou Xₐ-(CH₂)_{b}-(CF₂)_{c}-CF₃ ;
X est l'oxygène, S, N(R5),
a vaut zéro ou 1 ;
b vaut zéro, 1 ou 2 ;
c vaut zéro, 1, 2 ou 3 ;
R(5) est H, un radical alkyle ayant 1, 2, 3 ou 4 atomes de carbone ou -C_{d}H_{2d}R(6) ;
d vaut zéro, 1, 2, 3 ou 4 ;
R(6) est un radical cycloalkyle ayant 3, 4, 5, 6, 7 ou 8 atomes de carbone, phényle, biphénylyle ou naphtyle,
où les radicaux aromatiques phényle, biphénylyle ou naphtyle ne sont pas substitués ou sont substitués par 1 à 3 substituants choisis dans le groupe constitué par F, Cl, CF₃, méthyle, méthoxy et NR(7)R(8) ;
R(7) et R(8)
sont indépendamment H ou un radical alkyle ayant 1, 2, 3 ou 4 atomes de carbone ;
ou
R(1) est -SR(10), -O(R10) ou -CR(10)R(11)R(12) ;
R(10) est un radical -C_{f}H_{2f}-cycloalkyle avec 3, 4, 5, 6, 7 ou 8 atomes de carbone dans le cycle cycloalkyle, ou phényle,
où le radical phényle n'est pas substitué ou est substitué par 1 à 3 substituants choisis dans le groupe constitué par F, Cl, CF₃, CH₃, méthoxy, hydroxy, amino, méthylamino et diméthylamino ;
f vaut zéro, 1 ou 2 ;
R(11) et R(12)
sont indépendamment l'un de l'autre définis comme R(10) ou sont l'hydrogène ou un radical alkyle ayant 1, 2, 3 ou 4 atomes de carbone ;
ou
R(1) est un radical phényle, naphtyle, biphénylyle ou hétéroaryle avec 1, 2, 3, 4, 5, 6, 7, 8 ou 9 atomes de carbone, avec le dernier étant lié par un atome de C ou N du cycle,
qui sont dans chaque cas non substitués ou substitués par 1 à 3 substituants choisis dans le groupe constitué par F, Cl, CF₃, CH₃, méthoxy, hydroxy, amino, méthylamino et diméthylamino ;
ou
(R1) est -SR(13), -OR(13), -NHR(13), -NR(13)R(14), -CHR(13)R(15), -C[R(15)R(16)OH], -C≡CR(18), -C[R(19)]=CHR(18), -C[R(20)R(21)]ₖ-(CO)-[CR(22)R(23)]ₗ-R(24),
k vaut zéro, 1, 2, 3 ou 4 ;
l vaut zéro, 1, 2, 3 ou 4 ;
R(13) et R(14)
identiques ou différents, sont -(CH₂)_{g}-(CHOH)ₕ-(CH₂)ᵢ- (CHOH)ⱼ-R(17) ou -(CH₂)_{g}-O-(CH₂-CH₂O)ₕ-R(24) ;
R(17) est l'hydrogène ou le radical méthyle,
g, h et i identiques ou différents, valent zéro, 1, 2, 3 ou 4 ;
j vaut 1, 2, 3 ou 4 ;
R(15) et R(16) identiques ou différents, sont l'hydrogène, un radical alkyle ayant 1, 2, 3, 4, 5 ou 6 atomes de carbone, ou sont ensemble avec l'atome de carbone qui les porte un radical cycloalkyle avec 3, 4, 5, 6, 7 ou 8 atomes de carbone ;
R(18)
est un radical phényle,
qui est non substitué ou substitué par 1 à 3 substituants choisis dans le groupe constitué par F, Cl, CF₃, méthyle, méthoxy et NR(25)R(26) ;
R(25) et R(26)
sont H, un radical alkyle ayant 1, 2, 3 ou 4 atomes de carbone ;
ou
R(18)
est un radical hétéroaryle ayant 1, 2, 3, 4, 5, 6, 7, 8 ou 9 atomes de carbone,
qui est non substitué ou substitué comme le radical phényle ;
ou
R(18)
est un radical alkyle ayant 1, 2, 3, 4, 5 ou 6 atomes de carbone,
qui est non substitué ou substitué par 1 à 3 OH ;
ou
R(18)
est un radical cycloalkyle ayant 3, 4, 5, 6, 7 ou 8 atomes de carbone ;
R(19), R(20), R(21), R(22) et R(23)
identiques ou différents, sont l'hydrogène ou un radical méthyle ;
R(24)
est H, un radical alkyle ayant 1, 2, 3, 4, 5 ou 6 atomes de carbone, cycloalkyle ayant 3, 4, 5, 6, 7 ou 8 atomes de carbone ou -CₘH₂ₘ-R(18) ;
m vaut 1, 2, 3 ou 4 ;
un des deux substituants R(2) et R(3) est un radical hydroxyle ;
et
dans chaque cas l'autre des substituants R(2) et R(3) est défini comme R(1) ;
R(4) est un radical alkyle ayant 1, 2, 3 ou 4 atomes de carbone ; alcoxy ayant 1, 2, 3 ou 4 atomes de carbone, F, Cl, Br, I ou -(CH₂)ₙ-(CF₂)ₒ-CF₃;
n vaut zéro ou 1 ;
o vaut zéro ou 1 ;
ainsi que leurs sels acceptables du point de vue pharmacologique.

2. Composés de formule I selon la revendication 1, **caractérisés en ce que** :
R(1) est H, F, Cl, Br, I, CN, NO₂, un radical alkyle ayant 1, 2, 3, 4, 5, 6, 7 ou 8 atomes de carbone, alcoxy ayant 1, 2, 3, 4, 5, 6, 7 ou 8 atomes de carbone, cycloalkyle ayant 3, 4, 5, 6, 7 ou 8 atomes de carbone, cycloalcoxy ayant 3, 4, 5, 6, 7 ou 8 atomes de carbone ou Xₐ-(CH₂)_{c}-CF₃ ;
X est l'oxygène ;
a vaut zéro ou 1 ;
c vaut zéro, 1, 2 ou 3 ;
ou
R(1) est -SR(10), -O(R10) ;
R(10) est un radical -C_{f}H_{2f}-cycloalkyle avec 3, 4, 5, 6, 7 ou 8 atomes de carbone dans le cycle cycloalkyle, ou phényle,
où le radical phényle n'est pas substitué ou est substitué par 1 à 3 substituants choisis dans le groupe constitué par F, Cl, CF₃, CH₃, méthoxy, hydroxy, amino, méthylamino et diméthylamino ;
f vaut zéro ou 1;
ou
R(1) est un radical phényle, naphtyle, biphénylyle ou hétéroaryle avec 1, 2, 3, 4, 5, 6, 7, 8 ou 9 atomes de carbone, avec le dernier étant lié par un atome de C ou N du cycle,
qui sont dans chaque cas non substitués ou substitués par 1 à 3 substituants choisis dans le groupe constitué par F, Cl, CF₃, CH₃, méthoxy, hydroxy, amino, méthylamino et diméthylamino ;
ou
(R1) est -SR(13), -OR(13), -NHR(13), -NR(13)R(14), -C≡CR(18) ou -C[R(19)]=CHR(18) ;
R(13) et R(14)
identiques ou différents, sont -(CH₂)_{g}-(CHOH)ₕ-(CH₂)ᵢ-(CHOH)ⱼ-R(17) ou -(CH₂)_{g}-O-(CH₂-CH₂O)ₕ-R(24) ;
R(17) est l'hydrogène ou le radical méthyle,
g, h et i identiques ou différents, valent zéro, 1 ou 2 ;
j vaut 1 ou 2 ;
R(18)
est un radical phényle,
qui est non substitué ou substitué par 1 à 3 substituants choisis dans le groupe constitué par F, Cl, CF₃, méthyle, méthoxy et NR(25)R(26) ;
R(25) et R(26)
sont H ou un radical alkyle ayant 1, 2, 3 ou 4 atomes de carbone ;
ou
R(18)
est un radical hétéroaryle ayant 1, 2, 3, 4, 5, 6, 7, 8 ou 9 atomes de carbone,
qui est non substitué ou substitué comme le radical phényle ;
ou
R(18)
est un radical alkyle ayant 1, 2, 3, 4, 5 ou 6 atomes de carbone,
qui est non substitué ou substitué par 1 à 3 OH ;
ou
R(18)
est un radical cycloalkyle ayant 3, 4, 5, 6, 7 ou 8 atomes de carbone ;
R(19) est l'hydrogène ou le radical méthyle ;
un des deux substituants R(2) et R(3) est un radical hydroxyle ;
et
dans chaque cas l'autre des substituants R(2) et R(3) est défini comme R(1) ;
R(4) est un radical alkyle ayant 1 ou 2 atomes de carbone, méthoxy, F, Cl, Br, CN ou -(CH₂)ₙ-(CF₂)ₒ-CF₃;
n vaut zéro ou 1 ;
o vaut zéro ou 1.

3. Composés de formule I selon la revendication 1 ou 2, **caractérisés en ce que** :
R(1) est H, F, Cl, un radical alkyle ayant 1, 2, 3 ou 4 atomes de carbone, alcoxy ayant 1, 2, 3 ou 4 atomes de carbone, cycloalkyle ayant 5 ou 6 atomes de carbone, cycloalcoxy ayant 5 ou 6 atomes de carbone ou -Xₐ-(CH₂)_{c}-CF₃;
X est l'oxygène ;
a vaut zéro ou 1 ;
c vaut zéro ou 1 ;
ou
R(1) est -SR(10) ou -O(R10) ;
R(10) est un radical cycloalkyle ayant 4, 5 ou 6 atomes de carbone, ou phényle,
où le radical phényle n'est pas substitué ou est substitué par 1 à 3 substituants choisis dans le groupe constitué par F, Cl, CF₃, CH₃, méthoxy, hydroxy, amino, méthylamino et diméthylamino ;
ou
R(1) est un radical quinolyle, isoquinolyle, pyridyle,
qui sont reliés par un atome C ou un atome N du cycle ; et qui sont non substitués ou substitués par 1 à 3 substituants choisis dans le groupe constitué par F, Cl, CF₃, CH₃, méthoxy, hydroxy, amino, méthylamino et diméthylamino,
ou
(R1) est un radical phényle,
qui est non substitué ou substitué par 1 à 3 substituants choisis dans le groupe constitué par F, Cl, CF₃, CH₃, méthoxy, hydroxy, amino, méthylamino et diméthylamino,
ou
R(1) est -C≡CR(18) ;
R(18)
est un radical phényle ou cycloalkyle avec cinq ou six atomes de carbone ;
un des deux substituants R(2) et R(3) est un groupe hydroxyle ;
et dans chaque cas l'autre des substituants R(2) et R(3) est défini comme R(1) ;
R(4) est un radical méthyle, méthoxy, F, Cl ou CF₃.

4. Composé de formule I selon l'une ou plusieurs des revendications 1 à 3, **caractérisé en ce que** :
R(1) est H, F, Cl, un radical alkyle ayant 1, 2, 3 ou 4 atomes de carbone, alcoxy ayant 1, 2, 3 ou 4 atomes de carbone, cycloalkyle ayant 5 ou 6 atomes de carbone, cycloalcoxy ayant 5 ou 6 atomes de carbone ou CF₃ ;
un des deux substituants R(2) et R(3) est un groupe hydroxyle ;
et dans chaque cas l'autre des substituants R(2) et R(3) est défini comme R(1) ;
R(4) est un radical méthyle, méthoxy, F, Cl ou CF₃.

5. Composé de formule I selon la revendication 1, choisi dans le groupe constitué par : le chlorhydrate de 2-chloro-4-hydroxy-benzoylguanidine, le chlorhydrate de 2-chloro-4-hydroxy-5-iodo-benzoylguanidine, le chlorhydrate de 2-chloro-4-hydroxy-3,5-di-iodo-benzoylguanidine, le chlorhydrate de 2-chloro-4-hydroxy-5-trifluorométhyl-benzoylguanidine, le chlorhydrate de 4-hydroxy-2,5-trifluorométhyl-benzoylguanidine et le chlorhydrate de 4-hydroxy-2-méthyl-5-trifluorométhyl-benzoylguanidine.

6. Utilisation d'un composé I selon la revendication 1 pour la préparation d'un médicament pour le traitement ou la prophylaxie de maladies agissant sur les états ischémiques.

7. Utilisation d'un composé I selon la revendication 1 pour la préparation d'un médicament pour le traitement ou la prophylaxie de l'infarctus du myocarde.

8. Utilisation d'un composé I selon la revendication 1 pour la préparation d'un médicament pour le traitement ou la prophylaxie de l'angine de poitrine.

9. Utilisation d'un composé I selon la revendication 1 pour la préparation d'un médicament pour le traitement ou la prophylaxie des états ischémiques du coeur.

10. Utilisation d'un composé I selon la revendication 1 pour la préparation d'un médicament pour le traitement ou la prophylaxie des états ischémiques du système périphérique et central et de l'attaque cardiaque.

11. Utilisation d'un composé I selon la revendication 1 pour la préparation d'un médicament pour le traitement ou la prophylaxie des états ischémiques des organes périphériques et des poumons.

12. Utilisation d'un composé I selon la revendication 1 pour la préparation d'un médicament pour le traitement d'états de chocs.

13. Utilisation d'un composé I selon la revendication 1 pour la préparation d'un médicament pour l'emploi dans des opérations chirurgicales et des transplantations d'organes.

14. Utilisation d'un composé I selon la revendication 1 pour la préparation d'un médicament pour la conservation et le stockage d'organes à transplanter pour des transplantations chirurgicales.

15. Utilisation d'un composé I selon la revendication 1 pour la préparation d'un médicament pour le traitement de maladies pour lesquelles une prolifération cellulaire constitue une cause primaire ou secondaire.

16. Médicament, **caractérisé par** une quantité active d'un composé de formule I selon les revendications 1 à 5.
